**Europäisches Patentamt**

**European Patent Office**

**Office européen des brevets**

(19)

(11) Publication number : **0 477 135 A1**

# (12) EUROPEAN PATENT APPLICATION

(21) Application number : **91810677.4**

(22) Date of filing : **23.08.91**

(51) Int. Cl.⁵ : **A61K 9/50, A61K 9/52, A61K 9/68**

(30) Priority : **07.09.90 US 579753**

(43) Date of publication of application :
**25.03.92 Bulletin 92/13**

(84) Designated Contracting States :
**BE CH DE DK ES FR GB GR IT LI SE**

(71) Applicant : **WARNER-LAMBERT COMPANY**
**201 Tabor Road**
**Morris Plains New Jersey 07950 (US)**

(72) Inventor : **Tai, Anna W.**
**225 Longview Road**
**Bridgewater, New Jersey 08807 (US)**

(74) Representative : **Silbiger, Jakob, Dr.**
**c/o CAPSUGEL AG Fabrikmattenweg 2-4**
**CH-4144 Arlesheim (CH)**

(54) **Chewable spheroidal coated microcapsules and methods for preparing same.**

(57)    The present invention pertains to a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, wherein the coated microcapsule comprises (A) a microcapsule core comprising, in percentages by weight of the microcapsule core (a) a medicament present in an amount from about 1% to about 40%, (b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%, (c) an emulsifying agent present in an amount from about 0.5% to about 20%, (B) a first wax spray coating layer over the microcapsule core comprising, in percentages by weight of the first coating layer (a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%, (b) an emulsifying agent present in an amount from about 0.5% to about 20%, and (C) a second oil coating layer over the first coated microcapsule core comprising an edible vegetable oil having a melting point in the range from about 25°C. to about 90°C.

This invention pertains to novel chewable spheroidal coated microcapsules. The coated microcapsules comprise at least one spray congealed spheroidal microcapsule core under about 200 microns in diameter, a first wax spray coating layer over the microcapsule core, and a second oil coating layer over the first wax coated core. Therapeutically effective amounts of the coated microcapsules may be utilized in a wide variety of pharmaceutically acceptable carriers and confectionery bulking agents to prepare sustained release medicated compositions. This invention also relates to methods for preparing these chewable spheroidal coated microcapsules and the sustained release medicated compositions in which they may be used.

Sustained release compositions for the sequential or timed release of medicaments are well known in the art. Generally such compositions contain medicament particles, normally administered in divided doses two or more times daily, mixed with or covered by a coating material which is resistant to degradation or disintegration in the stomach and/or in the intestine for a selected period of time. Release of the medicament may occur by leaching, erosion, rupture, diffusion or similar actions, depending upon such factors as the nature and thickness of the coating material.

A frequently encountered problem in the field of chewable sustained release compositions is unsuitable particle size and shape. Sustained release particles, or microcapsules, having a size larger than about 850 microns are usually considered unsatisfactory for chewing because these particles are gritty and are easily broken during chewing thereby causing an off-taste and premature release of the medicament in the mouth. Spheroidal sustained release particles are generally preferred over nonspheroidal particles because these uniformly coated materials protect the medicament from premature release and release the medicament more uniformly. Large sustained release particles which must be ground to obtain small particles are usually not satisfactory for use in sustained release compositions because the particles are irregular, are not spheroidal and do not release the medicament uniformly.

United States patent no. 4,597,970, issued to Sharma et al. and assigned to Warner-Lambert Company, discloses a chewing gum composition having an agglomerated sweetener delivery system which comprises a sweetener core material and a hydrophobic matrix consisting essentially of (a) lecithin, (b) an edible material selected from the group consisting of (i) fatty acids, (ii) natural waxes, (iii) synthetic waxes, and (iv) mixtures thereof, and (c) at least one glyceride.

United States patent no. 4,291,062, issued to Leigh et al. and assigned to Phares Pharmaceutical Research N.V., discloses a pharmaceutical composition for protecting medicaments from moisture comprising a (a) medicament, (b) an inclusion compound of urea with an aliphatic compound, (c) an inert insoluble powder, and (d) an inert carrier. The medicament may be further protected from moisture by coating the medicament with a waxy material.

United States patent no. 4,788,180, issued to Bloch, discloses a pharmaceutical composition for administering magnesium which comprises a magnesium compound and a pharmaceutical carrier capable of slowly releasing the magnesium compound. The pharmaceutical carrier may be selected from the group consisting of a wax, a fatty acid, a fatty alcohol, and an ester.

United States patent no. 3,389,000, issued to Toyonaka et al. and assigned to Takeda Chemical Industries, Ltd., discloses a coated flavoring agent in granular form comprising a flavoring agent containing a nucleoside-5′-phosphate as a flavoring component and a coating agent of a fat melting at 55°C. to 85°C.

United States patent no. 3,146,167, issued to Lantz, Jr. et al. and assigned to Smith, Kline & French Laboratories, discloses a method for preparing a sustained release pharmaceutical pellet wherein the pellet comprises a medicament and a lipid material. The lipid material may be a wax, a fatty acid, alcohol or ester.

United States patent no. 4,572,833, issued to Pederson et al. and assigned to A/S Alfred Benzon, discloses a method for preparing a sustained release pharmaceutical composition wherein the composition comprises an active substance and a film-forming coating mixture. The coating mixture comprises a solvent, a film-forming substance dissolved in the solvent, and a hydrophobic substance. The hydrophobic may be a wax.

European patent application no. 222,411, filed November 14, 1986, to Taisho Pharmaceutical Co. Ltd., discloses a method for preparing a sustained release pharmaceutical composition which comprises applying a solution of binder and a fine powder of a hydrophobic solid material to the surface of solid particles containing a drug while the solid particles are being tumbled. The hydrophobic may be a higher alcohol, higher fatty acid, a higher fatty acid glycerin ester, an oil, a fat, a wax, or a higher hydrocarbon.

While the above sustained release compositions provide some degree of improved chewable sustained release activity, none of the above compositions are entirely satisfactory. Thus it would be advantageous to prepare a sustained release composition whereby the microcapsules formed are spheroidal to release the active ingredient uniformly and are of sufficiently small size such that they are not gritty and can be chewed without being broken. The present invention provides such improved chewable spheroidal coated microcapsules and sustained release medicated compositions without the disadvantages characteristic of previously known products. The present invention also provides methods for preparing these improved spheroidal coated microcapsules and the sustained release medicated compositions in which they may be employed.

The present invention pertains to a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, wherein the coated microcapsule comprises:

(A) a microcapsule core comprising, in percentages by weight of the microcapsule core:

(a) a medicament present in an amount from about 1% to about 40%;

(b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%;

(c) an emulsifying agent present in an amount from about 0.5% to about 20%;

(B) a first wax spray coating layer over the microcapsule core comprising, in percentages by weight of the first coating layer:

(a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%, and preferably present in an amount from about 80% to about 99.5%;

(b) an emulsifying agent present in an amount from abut 0.5% to about 20%; and

(C) a second oil coating layer over the first coated microcapsule core comprising an edible vegetable oil having a melting point in the range from about 25°C to about 90°C.

Applicant has found that by carefully controlling the concentration of the medicament and edible material having a melting point from about 25°C to about 100°C, and carefully controlling the conditions of spray congealing the mixture, spheroidal microcapsule cores of sufficiently small particle size suitable for use in chewable medicated compositions can be prepared. By forming the microcapsules by spray congealing, applicant's microcapsules are spheroidal and release the medicament very uniformly. Furthermore by carefully controlling the conditions for applying a first wax spray coating layer over the spheroidal microcapsule core, spheroidal coated microcapsules or spheroidal coated agglomerates of microcapsules, which contain a plurality of microcapsules in a single first coating, can be prepared. The emulsifying agent helps to increase the flexibility and reduce the brittleness of the microcapsule core and first coating layer. Finally, by applying an outer second oil coating layer over the first coated microcapsule core with a hard vegetable oil sufficiently flexible enough to seal the pores of the first coating without being brittle, improved spheroidal coated microcapsules suitable for use in chewable sustained release medicated compositions can be prepared. Applicant's coated microcapsules have improved delayed sustained release activity and can be employed for administering a medicament, normally administered in divided doses two or more times daily.

In accord with the present invention, chewable spheroidal coated microcapsules under about 850 microns in diameter may be prepared which comprise at least one spray congealed spheroidal microcapsule core under about 200 microns in diameter, a first wax spray coating layer over the core, and a second oil coating layer over the first wax coated core. The coated microcapsule comprises (A) a microcapsule core comprising, in percentages by weight of the microcapsule core (a) a medicament present in an amount from about 1% to about 40%, (b) an edible material having a melting point from about 25°C to about 100°C selected from the group consisting of (i) fatty acids having an iodine value from about 1 to about 10, (ii) natural waxes, (iii) synthetic waxes, and (iv) mixtures thereof, present in an amount from about 9.5% to about 98.5%, (c) an emulsifying agent present in an amount from about 0.5% to about 20%, (B) a first wax spray coating layer over the microcapsule core comprising, in percentages by weight of the first coating layer (a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60%, preferably about 80%, to about 99.5%, (b) an emulsifying agent present in an amount from about 0.5% to about 20%, and (C) a second oil coating layer over the first coated microcapsule core comprising an edible vegetable oil having a melting point in the range from about 25°C to about 90°C.

The coated microcapsule compositions may be utilized in a wide variety of pharmaceutically acceptable carriers and confectionery bulking agents to prepare medicated sustained release compositions. This invention also relates to methods for preparing these coated microcapsules and the medicated sustained release compositions in which they may be employed.

In a preferred embodiment, the spray congealed spheroidal microcapsule core (microcapsule) comprises in percentages by weight of the microcapsule core, (a) a medicament present in an amount from about 1% to about 40%, (b) an edible wax present in an amount from about 9.5% to about 98.5%, and (c) an emulsifying agent in an amount from about 0.5% to about 20%. In a more preferred embodiment, the spray congealed spheroidal microcapsule core comprises, in percentages by weight of the microcapsule core, (a) a medicament present in an amount from about 1% to about 35%, (b) an edible wax present in an amount from about 29.5% to about 98.5%, and (c) an emulsifying agent in an amount from about 0.5% to about 15%. In a most preferred embodiment, the spray congealed spheroidal microcapsule core comprises, in percentages by weight of the microcapsule core, (a) a medicament present in an amount from about 1% to about 30%, (b) an edible wax present in an amount from about 49.5% to about 98.5%, and (c) an emulsifying agent in an amount from about 0.5% to about 10%.

The medicaments (drugs, pharmaceuticals) of the present invention may be selected from a wide variety of water-soluble and water-insoluble drugs and their acid addition salts. Both organic and inorganic salts may be used provided the drug maintains its medicament value. Exemplary acid salts include hydrochloride, hydrobromide, orthophosphate, benzoate, maleate, tartrate, succinate, citrate, salicylate, sulfate and acetate.

The medicament may be selected from a wide range of therapeutic agents and mixtures of therapeutic agents which may be administered in sustained release or prolonged action form. Nonlimiting illustrative categories and specific examples of such medicaments include:

(a) Analgesics, such as acetylsalicylic acid, acetaminophen, ibuprofen, phenacetin, phenylbutazone, salicylamide, sodium salicylate, and meclofenamic acid;

(b) Anthelmintics, such as dithiazanine iodide and gardona;

(c) Antiasmatics, such as aminophylline, metaproterenol, epinephrine and theophylline;

(d) Anticholesterolemic and antilipid agents, such as gemfibrozil;

(e) Antiemetics, such as prochloroperazine dimaleate;

(f) Antihistamines, such as chlorpheniramine maleate, brompheniramine maleate, phenindamine tartrate, pyrilamine maleate, methapyrilene fumarate, doxylamine succinate, phenyltoloxamine citrate, diphenylhydramine hydrochloride, promethazine, terfenedine and triprolidine;

(g) Anti-inflammatory agents, such as isoxicam, meclophenamic acid and naproxen;

(h) Antinauseants, such as dimenhydrinate and meclizine;

(i) Antipyretics, such as N-acetyl-p-amino-phenol;

(j) Antitussives, such as dextromethorphan, dextromethorphan hydrobromide, noscapine, carbetapentane citrate, chlophedianol hydrochloride, codeine and diphenhydramine hydrochloride;

(k) Appetite suppressants, such as phenyl-propanolamine hydrochloride and caffeine;

(l) Cathartics, such as castor oil;

(m) Central nervous system stimulants, such as nicotine and caffeine;

(n) Decongestants, such as phenylephrine hydrochloride, phenylpropanolamine hydrochloride, pseudoephedrine hydrochloride, pseudoephedrine hydrobromide, pseudoephedrine sulfate and ephedrine;

(o) Expectorants, such as guaifenesin and glycerol guaiacolate;

(p) Laxatives, such as phenolphthalein, danthron, pamabrom and bisocadyl;

(q) Nutritional supplements, including vitamins and minerals, such as ascorbic acid, niacin, pantothenic acid, vitamin $B_6$, thiamine hydrochloride, riboflavin, potassium iodide, potassium chloride, cupric sulfate and ferrous sulfate; and

(r) Various alkaloids, such as codeine phosphate, codeine sulfate and morphine.

In a preferred embodiment, the medicament is a water-soluble medicament selected from the group consisting of dextromethorphan, dextromethorphan hydrobromide, pseudoephedrine, pseudoephedrine sulfate, pseudoephedrine hydrochloride, pseudoephedrine hydrobromide, chlorpheniramine maleate, guaifenesin,diphenhydramine hydrochloride, and mixtures thereof. In a more preferred embodiment the medicament is a water-soluble medicament selected from the group consisting of pseudoephedrine sulfate,diphenhydramine hydrochloride, chlorpheniramine maleate, and mixtures thereof.

The particle size of the medicament in the present invention will be a size sufficient such that when the medicament is mixed with the edible material and the emulsifying agent components of the microcapsule core in the proportions set out above, spray congealed spheroidal microcapsule cores under about 200 microns in diameter, preferably under about 175 microns, and most preferably under about 150 microns, can be obtained. In general, the particle size of the medicament will be under about 50 microns, preferably under about 35 microns, and more preferably under about 25 microns.

The medicament of the present invention may be used in many distinct physical forms well known in the pharmaceutical art to provide an initial dosage of the medicament and/or a further time-release form of the medicament. Without being limited thereto, such physical forms include free forms and encapsulated forms, and mixtures thereof.

The amount of medicament drug or its acid addition salt used in the present invention may vary depending upon the therapeutic dosage recommended or permitted for the particular medicament. In general, the amount of medicament present is the ordinary dosage required to obtain the desired result. Such dosages are known to the skilled practitioner in the medical arts and are not a part of the present invention.

The edible material in the first wax spray coating layer is a material which has a melting point in the range from about 25°C to about 100°C, preferably from about 35°C to about 100°C, and more preferably from about 45°C to about 100°C. The melting point of the edible material should be within the recited range because the melting point of the final coated microcapsule product will be greatly affected by the fat or wax constituent.

The materials useful in the first wax spray coating layer are selected from the group consisting of fatty acids, natural waxes, synthetic waxes, and the like, and mixtures thereof. Fatty acids are carboxylic acids derived from or contained in an animal or vegetable fat or oil. Fatty acids are composed of a chain of alkyl groups containing from 4 to 22 carbon atoms and are characterized by a terminal carboxyl group. Waxes are low-melting organic mixtures or compounds having a high molecular weight, are solid at room temperature and generally are similar in composition to fats and oils except that waxes contain no glycerides. Waxes may be hydrocarbons or esters of fatty acids and alcohols. Fatty acids and waxes are both classified as lipids.

The fatty acids useful in the present invention are acids which have an iodine value from about 1 to about 10. The iodine value is a means of determining the degree of unsaturation in a fat or oil. The measurement of iodine values is determined by known titrating methods and is reported in terms of centigrams of iodine absorbed per gram of fat or oil sample titrated. (See "Bailey's Industrial Oil and Fat Products," Vol. 2, 4th Ed., Swern, Daniel ed., pp. 436-438 (1982)). Hence the fatty acids useful in the present invention have an iodine value from about 1 centigram to about 10 centigrams.

Fatty acids useful in the present invention are selected from the group consisting of hydrogenated palm oil, hydrogenated palm kernel oil, hydrogenated peanut oil, hydrogenated rapeseed oil, hydrogenated rice bran oil, hydrogenated soybean oil, hydrogenated cottonseed oil, hydrogenated sunflower oil, hydrogenated castor oil, and the like, and mixtures thereof. Other fatty acids include, for example, decenoic acid, docosanoic acid, stearic acid, palmitic acid, lauric acid, myristic acid, and the like, and mixtures thereof. The preferred fatty acids are selected from the group consisting of hydrogenated palm oil, hydrogenated castor oil, hydrogenated cottonseed oil, stearic acid, palmitic acid, and mixtures thereof. The most preferred fatty acid is stearic acid.

Waxes useful in the present invention include natural waxes, such as animal waxes, vegetable waxes, and petroleum waxes (i.e., paraffin waxes, microcrystalline waxes, petrolatum waxes, mineral waxes), and synthetic waxes which are edible and have a melting point within the range from about 25°C to about 100°C. Specific examples of useful waxes are spermaceti wax, carnauba wax, Japan wax, bayberry wax, flax wax, beeswax, Chinese wax, shellac wax, lanolin wax, sugarcane wax, candelilla wax, paraffin wax, microcrystalline wax, petrolatum wax, carbowax, and the like, and mixtures thereof. Mixtures of these waxes with the fatty acids set out above may also be used. The preferred waxes are selected from the group consisting of carnauba wax, bees wax, glyceryl tripalmitate, glyceryl monostearate, paraffin wax, microcrystalline wax, glyceryl distearate, glyceryl tristearate (Dynassan-118), and mixtures thereof. The most preferred waxes are carnauba wax, bees wax, glyceryl tristearate, glyceryl monostearate, and paraffin wax.

The wax may also be an ester of a fatty acid having from about 12 to about 31 carbon atoms and a fatty alcohol having from about 12 to about 31 carbon atoms, the ester having a carbon atom content from about 24 to about 62 carbon atoms. Examples of such fatty acid esters are myricyl palmitate, ceryl palmitate, ceryl cerotate, myricyl melissate, stearyl palmitate, stearyl myristate, lauryl laurate, and the like, and mixtures thereof. The preferred fatty acid esters are selected from the group consisting of stearyl palmitate, stearyl myristate, and mixtures thereof.

The wax may also be a monoglyceryl ester, diglyceryl ester, or triglyceryl ester (glycerides) which is an ester formed from a fatty acid having from about 10 to about 22 carbon atoms and glycerol, wherein one or more of the hydroxyl groups of glycerol is substituted by a fatty acid. Examples of useful glycerides include glyceryl monostearate, glyceryl distearate, glyceryl tristearate, glyceryl dipalmitate, glyceryl tripalmitate, glyceryl monopalmitate, glyceryl dilaurate, glyceryl trilaurate, glyceryl monolaurate, glyceryl didocosanoate, glyceryl tridocosanoate, glyceryl monodocosanoate, glyceryl monocaproate, glyceryl dicaproate, glyceryl tricaproate, glyceryl monomyristate, glyceryl dimyristate, glyceryl trimyristate, glyceryl monodecenoate, glyceryl didecenoate, glyceryl tridecenoate, and the like, and mixtures thereof. The preferred glycerides are selected from the group consisting of glyceryl monostearate, glyceryl distearate, glyceryl tristearate, and mixtures thereof.

The emulsifying agent in the microcapsule core in this invention helps to disperse immiscible components into a single stable system, to form spheroidal coated microcapsules, and to increase the flexibility and reduce the brittleness of the microcapsule core. Emulsifying agents (surfactants, wetting agents) are compounds which, inter alia, reduce interfacial tension between a liquid and a solid. Emulsifying agents useful in this invention include acetylated monoglycerides (Cetodan-700), stearic acid, oleic acid, polyethylene glycol, glyceryl monostearate, lecithin, fatty acid monoglycerides (Dimodan-PVK), diglycerides, propylene glycol monostearate, and the like, and mixtures thereof. The preferred emulsifying agents are selected from the groups consisting of glyceryl monostearate, acetylated monoglycerides (Cetodan-700), fatty acid monoglycerides (Dimodan-PVK), stearic acid, and the like, and mixtures thereof. The more preferred emulsifying agent is selected from the group consisting of acetylated monoglycerides (Cetodan-700) and fatty acid monoglycerides (Dimodan-PVK).

The microcapsule cores of the present invention have a diameter under about 200 microns, preferably under about 175 microns, and most preferably under about 150 microns. The spray congealed microcapsule cores formed in the present invention have an approximate elliptical or spherical shape and are spheroidal, that is, the microcapsules formed resemble spheres.

In a preferred embodiment, the first wax spray coating layer over the microcapsule core comprises, in percentages by weight of the first coating layer, a fatty acid or wax edible material present in the range from about 60% to about 99.5%, more preferably from about 70% to about 99.5%, and most preferably from about 80% to about 99.5%. These amounts are necessary to adequately coat the microcapsule core and provide sustained release properties. Preferably, the emulsifying agent in the first wax spray coating layer is present, in percentages by weight of the first coating layer, in the range from about 0.5% to about 20%, more preferably from about 0.5% to about 15%, and most preferably from about 0.5% to about 10%.

The edible materials and emulsifying agents described above as suitable for use in the spray congealed microcapsule core are also suitable for use in the first wax spray coating layer.

The first wax spray coated microcapsule cores of the present invention have a diameter under about 600 microns, preferably under about 500 microns, and most preferably under about 450 microns. The first wax spray coated microcapsule cores formed in the present invention have an approximate elliptical or spherical shape and are spheroidal.

The edible vegetable oil in the second oil coating layer is an oil which has a melting point in the range from about 25°C. to about 90°C., preferably from about 30°C. to about 80°C., and more preferably from about 35°C. to about 70°C. The melting point of the edible vegetable oil should be within the recited range because the vegetable oil must be sufficiently flexible to seal the pores of the first coating and must not be brittle. The final coated microcapsule product will be greatly affected by the melting point of the vegetable oil. The vegetable oils useful in the present invention are selected from the group consisting of hard palm oil (mp. 58-62°C.), partially hydrogenated castor oil (mp. 86-88°C.), partially hydrogenated cotton seed oil (mp. 46-48°C.), partially hydrogenated soybean oil (mp. 67-70°C.), and blends of partially hydrogenated palm kern oil, partially hydrogenated cotton seed oil, and partially hydrogenated soybean seed oil (mp. 38-40°C., such as Paramount fat C), and the like, and mixtures thereof. In a more preferred embodiment, the vegetable oils are selected from the group consisting of mixtures of hard palm oil, and a blend of partially hydrogenated palm kern oil, partially hydrogenated cotton seed oil, and partially hydrogenated soybean seed oil, and mixtures thereof. In a most preferred embodiment, the vegetable oils are a mixture of hard palm oil, and a blend of partially hydrogenated palm kern oil, partially hydrogenated cotton seed oil, and partially hydrogenated soybean seed oil, in a ratio of 2:1, respectively.

The weight ratio of microcapsule core to first wax spray coating layer to second oil coating layer is the ratio containing sufficient coating layer to prevent potential premature release of the medicament from the microcapsule core without forming a composition so large so as to be therapeutically unsuitable for use in chewable compositions. In general, the weight ratio of the microcapsule core to first wax spray coating layer to second oil coating layer is from about 1:1:0.5 to about 1:3:3, preferably from about 1:1:1 to about 1:2:2, and more preferably from about 1:1:2 to about 1:2:1, respectively.

The chewable spheroidal coated microcapsules of the present invention, having a first wax spray coating and a second oil coating, have a diameter under about 850 microns, preferably under about 700 microns, and most preferably under about 600 microns. The spheroidal coated microcapsules may also be agglomerates of coated microcapsules which may contain one or more core microcapsule gathered into a cluster under the coating layer. The terms "coated microcapsules" and "agglomerates of coated microcapsules" are used interchangeably herein.

In a preferred embodiment, the chewable spheroidal coated microcapsule comprises:

(a) a microcapsule core comprising the following ingredients:

| INGREDIENT | PERCENTAGE RANGE BY WEIGHT OF MICROCAPSULE CORE |
|---|---|
| Pseudoephedrine sulfate | 1-40 |
| Glyceryl Tristearate | 1-95.9 |
| Carnauba Wax | 1-95.9 |
| Acetylated monoglycerides | 1-10 |
| Fatty acid monoglycerides | 1-10 |
| Aluminum stearate | 0.1-5 |

(b) a first wax spray coating layer comprising the following ingredients:

| INGREDIENT | PERCENTAGE RANGE BY WEIGHT OF FIRST COATING LAYER |
|---|---|
| Carnauba wax | 1-97 |
| Glyceryl Tristearate | 1-97 |
| Paraffin 150 | 1-10 |
| Fatty acid monoglycerides | 1-10 |

(C) a second oil coating layer comprising the following ingredients:

| INGREDIENT | PERCENTAGE RANGE BY WEIGHT OF SECOND COATING LAYER |
|---|---|
| Blend of Partially hydrogenated cotton seed oil Partially hydrogenated soybean seed oil Partially hydrogenated palm kern oil | 10-90 |
| Palm Oil | 10-90 |

The present invention is also directed at methods for preparing the spray congealed spheroidal microcapsule cores. The spray congealed microcapsule cores are prepared by mixing at low shear the fatty acid or wax edible material with the emulsifying agent at a temperature from about 80°C. to about 100°C. until a homogeneous mixture is obtained. The medicament is then added to this mixture and mixed to uniformly disperse the medicament in the mixture. The dispersion is then fed into a heat controlled spray nozzle and spray congealed. The term spray congealing as used herein refers to the solidification of the atomized liquid droplets which cool and solidify upon contacting the cooler temperature of the surrounding atmosphere. The nozzle pressure is regulated to control the particle droplet size. The droplets cool and congeal once they are emitted from the nozzle and contact the cooler environment. The resulting dry particles or agglomerates having an approximate elliptical or spherical (spheroidal) shape.

The microcapsule cores of the present invention are spray congealed under controlled conditions such that spheroidal microcapsules under about 200 microns in diameter are formed. The microcapsules of the present invention may be spray congealed using standard techniques and equipment known to those skilled in the art. The exact conditions for spray congealing will vary with the particular apparatus selected and are readily determined by those skilled in the art without the need for undue experimentation. Spray congealing apparatus is well known in the arts and therefore the selection of the specific apparatus will be apparent to the artisan. The microcapsules may then be screened to the desired mesh size.

The present invention is also directed at methods for coating the spray congealed microcapsule cores with the first wax spray coating layer. The first microcapsules may be spray coated with the edible material and emulsifying agent set out above by methods well known in the art such that spheroidal first wax coated microcapsules under about 600 microns in diameter are formed. These methods include, for example, the fluidized bed granulation technique, the spray congealing technique, and the pulverization technique.

In a preferred embodiment, the spray congealed microcapsules are spray coated with the first wax spray coating layer by the fluidized bed granulation technique. In this embodiment, the spheroidal spray congealed microcapsules in the present invention are spray (film) coated in a fluid bed dryer by suspending the microcapsules in a stream of air or strong upward air current and passing the stream through a zone of finely atomized droplets of the first coating layer mixture (encapsulant), after which the coated particles pass out of the upward stream and pass downward in a fluidized condition countercurrent to a flow of heated fluidized gas whereupon they are dried, and may re-enter the upward-moving coating zone for a subsequent discreet coating application. The microcapsules of the present invention are first spray coated under controlled conditions such that spheroidal coated microcapsules under about 600 microns in diameter are formed.

The microcapsules of the present invention may be spray coated with the first wax spray coating layer using standard techniques and equipment known to those skilled in the art. The exact conditions for spray coating will vary with the particular fluid bed apparatus selected and are readily determined by those skilled in the art without the need for undue experimentation. Fluid bed apparatus is well known in the arts and therefore the selection of the specific apparatus will be apparent to the artisan.

In one preferred embodiment, the method and apparatus employed is known as the Wurster Process. The Wurster Process and its associated apparatus are disclosed in detail, for example, in United States patents no. 3,089,824, 3,117,027, 3,196,827, 3,241,520, and 3,253,944, which disclosures are incorporated herein by reference.

In this preferred embodiment, the apparatus is a Versa-Glatt model GPCG 1 fluidized bed apparatus with a Wurster column. The exact conditions for spray coating will vary with the particular apparatus selected and are readily determined by those skilled in the art without the need for undue experimentation. In general, the spray rate during the coating is preferably from about 2 ml/min. to about 10 ml/min., more preferably from about 2 ml/min. to about 7 ml/min., and most preferably from about 2 ml/min. to about 6 ml/min. The fluidizing (atomizing) air pressure is preferably from about 1 atmosphere to about 5 atmospheres, more preferably from about 1 atmosphere to about 4.5 atmospheres, and most preferably from about 1 atmosphere to about 4 atmospheres. The nozzle setting is preferably from about 0.8 mm to about 2 mm, more preferably from about 0.8 mm to about 1.5 mm, and most preferably from about 0.8 mm to about 1.2 mm. The inlet temperature is preferably from about 35°C. to about 65°C., more preferably from about 35°C. to about 60°C., and most preferably from about 40°C. to about 55°C. The outlet temperature is preferably from about 20°C. to about 50°C., more preferably from about 25°C. to about 50°C., and most preferably from about 30°C. to about 50°C.

In an alternative but less preferred embodiment, the spray congealed spheroidal microcapsules are coated with the first wax spray coating layer by the spray congealing technique. In this embodiment, the first coating layer is prepared by mixing at low shear the fatty acid or wax edible material with the emulsifying agent at temperatures from about 75°C. to about 95°C. until a homogeneous mixture is obtained. The microcapsule core material is then added to this first wax spray coating layer mixture and mixed to uniformly disperse the material in the mixture. The dispersion is then fed into a heat controlled spray nozzle and spray congealed. The microcapsules of the present invention are spray congealed under controlled conditions such that spheroidal coated microcapsules under about 600 microns in diameter are formed. Spray congealing techniques and equipment have been set out above for the preparation of the microcapsule core. The exact conditions for spray congealing will vary with the particular apparatus selected and are readily determined by those skilled in the art without the need for undue experimentation.

In another alternative but less preferred embodiment, the spray congealed spheroidal microcapsules are coated with the first wax spray coating layer by the pulverization technique. A uniform mixture of spray congealed microcapsules and melted first coating layer mixture of edible material and emulsifying agent are cooled in sheets and pulverized to a particle size less than about 600 microns.

The present invention is also directed at methods for coating the first wax spray coated microcapsules with a second oil coating layer. The first wax spray coated microcapsules may be coated with the vegetable oils set out above as useful in the second oil coating layer by methods well known in the art such that chewable first and second spheroidal coated microcapsule under about 850 microns in diameter are formed. These methods include, for example, the fluidized bed granulation technique, the spray congealing technique, and the pulverization technique.

Preferably, the first wax spray coated microcapsules are coated with the second oil coating layer by melting the vegetable oil to be employed as the second oil coating layer and then admixing the coated microcapsule cores with the melted second coating layer to form a homogeneous mixture of the chewable spheroidal coated microcapsule of the present invention.

In a preferred embodiment, the present invention is directed at a method for preparing a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, which comprises the steps of:

(A) providing the following ingredients of the microcapsule core, in percentages by weight of the microcapsule core:

(a) a medicament present in an amount from about 1% to about 40%;

(b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%; and

(c) an emulsifying agent present in an amount from about 0.5% to about 20%; and

(B) mixing and heating the microcapsule core ingredients from step (A) to prepare a homogeneous mixture;

(C) feeding the mixture from step (B) into a heat controlled pressure spray nozzle and atomizing the mixture under controlled conditions such that spheroidal spray congealed core microcapsules under about 200 microns in diameter are formed;

(D) providing the following ingredients of the first wax spray coating layer, in percentages by weight of the first coating layer:

(a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(E) preparing a homogeneous mixture of the first coating layer ingredients from step (D);

(F) coating the spray congealed core microcapsules from step (C) with the first coating layer mixture from step (E) in a fluid bed dryer by suspending the microcapsule cores in a stream of air passing through a zone of the first coating layer mixture under controlled conditions such that spheroidal first coated microcapsules under about 600 microns in diameter are formed;

(G) providing a second oil coating layer comprising an edible vegetable oil having a melting point in the range from about 25°C. to about 90°C.;

(H) mixing and melting the second oil coating layer ingredients from step (G) to prepare a homogeneous mixture; and

(I) admixing the spheroidal first coated microcapsules from step (F) with the melted second layer mixture from step (H) to prepare a homogeneous mixture of chewable spheroidal coated microcapsules under about 850 microns in diameter.

Once prepared, the chewable spheroidal coated microcapsules may be stored for future use or may be formulated with conventional additives such as pharmaceutically acceptable carriers and confectionery bulking agents to prepare a wide variety of chewable medicated sustained release compositions to suit particular applications.

An important aspect of the present invention includes a hard or soft confectionery composition incorporating the inventive chewable spheroidal coated microcapsule compositions and a method for preparing the hard or soft confections. In this form of the invention, the chewable spheroidal coated microcapsules are incorporated in a pharmaceutically acceptable carrier such as a confectionery bulking agent, along with various additives. The confectionery may be in the form of a lozenge, tablet, toffee, nougat, suspension, chewy candy, and the like. In a preferred embodiment, the confectionery is a nougat. The pharmaceutically acceptable carriers may be prepared from a wide range of materials. Without being limited thereto, such materials include diluents, binders and adhesives, lubricants, disintegrants, coloring agents, bulking agents, flavoring agents, sweetening agents and miscellaneous materials such as buffers and adsorbents in order to prepare a particular medicated chewable confection.

The preparation of confectionery formulations is historically well known and has changed little through the years. Confectionery items have been classified as either "hard" confectionery or "soft" confectionery. The chewable medicated compositions of the present invention can be incorporated into confectionery compositions by admixing the inventive compositions into conventional hard and soft confections.

As used herein, the term confectionery material means a product containing a bulking agent selected from a wide variety of materials such as sugar, corn syrup, and the like, and in the case of sugarless bulking agents, sugar alcohols such as sorbitol and mannitol and the like, and mixtures thereof. Confectionery material may include such exemplary substances as lozenges, tablets, toffee, nougat, suspensions, chewy candy, chewing gum and the like. The bulking agent is present in a quantity sufficient to bring the total amount of confectionery composition to 100%.

Lozenges are flavored medicated dosage forms intended to be sucked and held in the mouth. Lozenges may be in the form of various shapes such as flat, circular, octagonal and biconvex forms. The lozenge bases are generally in two forms: hard, boiled candy lozenges and compressed tablet lozenges.

Hard boiled candy lozenges may be processed and formulated by conventional means. In general, a hard boiled candy lozenge has a base composed of a mixture of sugar and other carbohydrate bulking agents kept in an amorphous or glassy condition. This amorphous or glassy form is considered a solid syrup of sugars generally having from about 0.5% to about 1.5% moisture. Such materials normally contain up to about 92% corn syrup, up to about 55% sugar and from about 0.1% to about 5% water, by weight of the final composition. The syrup component is generally prepared from corn syrups high in fructose, but may include other materials.

Further ingredients such as flavoring agents, sweetening agents, acidulants, coloring agents and the like may also be added.

Boiled candy lozenges may also be prepared from non-fermentable sugars such as sorbitol, mannitol, and hydrogenated corn syrup. Typical hydrogenated corn syrups are Lycasin, a commercially available product manufactured by Roquette Corporation, and Hystar, a commercially available product manufactured by Lonza, Inc. The candy lozenges may contain up to about 95% sorbitol, a mixture of sorbitol and mannitol in a ratio from about 9.5:0.5 up to about 7.5:2.5, and hydrogenated corn syrup up to about 55%, by weight of the solid syrup component.

Boiled candy lozenges may be routinely prepared by conventional methods such as those involving fire cookers, vacuum cookers, and scraped-surface cookers also referred to as high speed atmospheric cookers.

Fire cookers involve the traditional method of making a boiled candy lozenge base. In this method, the desired quantity of carbohydrate bulking agent is dissolved in water by heating the agent in a kettle until the bulking agent dissolves. Additional bulking agent may then be added and the cooking continued until a final temperature of 145°C to 156°C is achieved. The batch is then cooled and worked as a plastic-like mass to incorporate additives such as flavoring agents, coloring agents and the like.

A high-speed atmospheric cooker uses a heat-exchanger surface which involves spreading a film of candy on a heat exchange surface, the candy is heated to 165°C to 170°C in a few minutes. The candy is then rapidly cooled to 100°C to 120°C and worked as a plastic-like mass enabling incorporation of the additives, such as flavor agents, coloring agents and the like.

In vacuum cookers, the carbohydrate bulking agent is boiled at a temperature from about 125°C to about 132°C, vacuum is applied and additional water is boiled off without extra heating. When cooking is complete, the mass is a semi-solid and has a plastic-like consistency. At this point, flavoring agents, coloring agents, and other additives are admixed in the mass by routine mechanical mixing operations.

The optimum mixing required to uniformly mix the flavoring agents, coloring agents and other additives during conventional manufacturing of boiled candy lozenges is determined by the time needed to obtain a uniform distribution of the materials. Normally, mixing times of from about 4 to about 10 minutes have been found to be acceptable.

Once the boiled candy lozenge has been properly tempered, it may be cut into workable portions or formed into desired shapes. A variety of forming techniques may be utilized depending upon the shape and size of the final product desired. A general discussion of the composition and preparation of hard confections may be found in H.A. Lieberman, Pharmaceutical Dosage Forms: Tablets, Volume 1 (1980), Marcel Dekker, Inc., New York, N.Y. at pages 339 to 469, which disclosure is incorporated herein by reference.

The apparatus useful in accordance with the present invention comprises cooking and mixing apparatus well known in the confectionery manufacturing arts, and therefore the selection of the specific apparatus will be apparent to the artisan.

In contrast, compressed tablet confections contain particulate materials and are formed into structures under pressure. These confections generally contain sugars in amounts up to about 95%, by weight of the composition, and typical tablet excipients such as binders and lubricants as well as flavoring agents, coloring agents and the like.

In addition to hard confectionery materials, the lozenges of the present invention may be made of soft confectionery materials such as those contained in nougat. The preparation of soft confections, such as nougat, involves conventional methods, such as the combination of two primary components, namely (1) a high boiling syrup such as a corn syrup, hydrogenated starch hydrolysate or the like, and (2) a relatively light textured frappe, generally prepared from egg albumin, gelatin, vegetable proteins, such as soy derived compounds, sugarless milk derived compounds such as milk proteins, and mixtures thereof. The frappe is generally relatively light, and may, for example, range in density from about 0.5 to about 0. 7 grams /cc.

The high boiling syrup, or "bob syrup" of the soft confectionery is relatively viscous and has a higher density than the frappe component, and frequently contains a substantial amount of carbohydrate bulking agent such as a hydrogenated starch hydrolysate. Conventionally, the final nougat composition is prepared by the addition of the "bob syrup" to the frappe under agitation, to form the basic nougat mixture. Further ingredients such as flavoring agents, additional carbohydrate bulking agents, coloring agents, preservatives, medicaments, mixtures thereof and the like may be added thereafter also under agitation. A general discussion of the composition and preparation of nougat confections may be found in B.W. Minifie, Chocolate, Cocoa and Confectionery: Science and Technology, 2nd edition, AVI Publishing Co., Inc., Westport, Conn. (1980), at pages 424-425, which disclosure is incorporated herein by reference.

The procedure for preparing the soft confectionery involves known procedures. In general, the frappe component is prepared first and thereafter the syrup component is slowly added under agitation at a temperature of at least about 65°C, and preferably at least about 100°C. The mixture of components is continued to be mixed to form a uniform mixture, after which the mixture is cooled to a temperature below 80° C, at which point, the flavoring agent may be added. The mixture is further mixed for an additional period until it is ready to be removed and formed into suitable confectionery shapes.

Chewable medicated candy is prepared by procedures similar to those used to make soft confectionery. In a typical procedure, a boiled sugar-corn syrup blend is formed to which is added a frappe mixture. The boiled sugar-corn syrup blend may be prepared from sugar and corn syrup blended in parts by weight ratio of about 90:10 to about 10:90. The sugar-corn syrup blend is heated to temperatures above about 120°C. to remove water and to form a molten mass. The frappe is generally prepared from gelatin, egg albumin, milk proteins such as casein, and vegetable proteins such as soy protein, and the like, which is added to a gelatin solution and rapidly mixed at ambient temperature to form an aerated sponge like mass. The frappe is then added to the molten candy mass and mixed until homogeneous at temperatures between about 65°C and about 120°C.

The chewable spheroidal coated microcapsule compositions of the instant invention can then be added to the homogeneous mixture as the temperature is lowered to about 50°C-65°C whereupon additional ingredients can then be added such as flavoring agents and coloring agents. The formulation is further cooled and formed into pieces of desired dimensions.

A general discussion of the lozenge and chewable tablet forms of confectionery may be found in H.A. Lieberman and L. Lachman, Pharmaceutical Dosage Forms: Tablets Volume 1, Marcel Dekker, Inc., New York, N.Y. at pages 289 to 466, which disclosure is incorporated herein by reference.

In accordance with this invention, therapeutically effective amounts of the chewable spheroidal coated microcapsule compositions of the present invention may be admixed into the hard and soft confections. These amounts are readily determined by those skilled in the art without the need for undue experimentation. The exact amount of the chewable spheroidal coated microcapsules employed in the hard and soft confections will vary with the particular medicament selected. In a preferred embodiment, the chewable spheroidal coated microcapsules are present in the hard and soft confection compositions in percentages by weight in an amount from about 5% to about 50%, more preferably from about 10% to about 40%, and most preferably, in an amount from about 10% to about 30%. The pharmaceutically acceptable carrier and optional additives are present in a quantity sufficient to bring the total amount of hard and soft confection composition to 100%.

In a preferred embodiment, the present invention is directed at a chewable sustained release medicated composition which comprises a pharmaceutically acceptable carrier and a therapeutically effective amount of a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, wherein the coated microcapsule comprises:

(A) a microcapsule core comprising, in percentages by weight of the microcapsule core:

(a) a medicament present in an amount from about 1% to about 40%;

(b) an edible material having a melting point from about 25°C. to about 100°C. selected from the group consistingof (i) fatty acids having an iodine value from about 1 to about 10, (ii) natural waxes, (iii) synthetic waxes, and (iv) mixtures thereof, present in an amount from about 9.5% to about 98.5%;

(c) an emulsifying agent present in an amount from about 0.5% to about 20%;

(B) a first wax spray coating layer over the microcapsule core comprising, in percentages by weight of the first coating layer:

(a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(C) a second oil coating layer over the first coated microcapsule core comprising an edible vegetable oil having a melting point in the range from about 25°C. to about 90°C.

The present invention extends to methods of making the improved chewable medicated hard and soft confection compositions. The medicated microcapsule compositions may be incorporated into otherwise conventional hard or soft confection compositions using standard techniques and equipment known to those skilled in the art.

For example, the microcapsule cores coated with the first wax spray coating layer can be admixed with the melted second coating layer to form a homogeneous mixture of the chewable spheroidal coated microcapsule of the present invention. The homogeneous mixture may then be admixed with a pharmaceutical carrier to form a sustained release medicated composition.

In a preferred embodiment, the present invention is directed at a method for preparing a chewable sustained release medicated composition which comprises a pharmaceutically acceptable carrier and a therapeutically effective amount of a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, which comprises the steps of:

(A) providing the following ingredients of the microcapsule core, in percentages by weight of the microcapsule core:

(a) a medicament present in an amount from about 1% to about 40%;

(b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%; and

(c) an emulsifying agent present in an amount from about 0.5% to about 20%; and

(B) mixing and heating the microcapsule core ingredients from step (A) to prepare a homogeneous mixture;

(C) feeding the mixture from step (B) into a heat controlled pressure spray nozzle and atomizing the mixture under controlled conditions such that spheroidal spray congealed core microcapsules under about 200 microns in diameter are formed;

(D) providing the following ingredients of the first wax spray coating layer, in percentages by weight of the first coating layer:

(a) an edible material having a melting point from about 25° C. to about 100° C. selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(E) preparing a homogeneous mixture of the first coating layer ingredients from step (D);

(F) coating the spray congealed core microcapsules from step (C) with the first coating layer mixture from step (E) in a fluid bed dryer by suspending the microcapsule cores in a stream of air passing through a zone of the first coating layer mixture under controlled conditions such that spheroidal first coated microcapsules under about 600 microns in diameter are formed;

(G) providing a second oil coating layer comprising an edible vegetable oil having a melting point in the range from about 25°C. to about 90°C.;

(H) mixing and melting the second oil coating layer ingredients from step (G) to form a homogeneous mixture;

(I) admixing the first coated microcapsules from step (F) with the melted second layer mixture from step (H) to prepare a homogeneous mixture of chewable spheroidal coated microcapsules under about 850 microns in diameter; and

(J) admixing the microcapsules from step (I) having a first coating layer and a second coating layer with the pharmaceutical carrier to form a chewable sustained release medicated composition.

The apparatus useful in accordance with the present invention comprises cooking and mixing apparatus well known in the confectionery manufacturing arts, and therefore the selection of the specific apparatus will be apparent to the artisan.

Throughout this application, various publications have been referenced. The disclosures in these publications are incorporated herein by reference in order to more fully describe the state of the art.

The present invention is further illustrated by the following examples which are not intended to limit the effective scope of the claims. All parts and percentages in the examples and throughout the specification and claims are by weight of the final composition unless otherwise specified.

## EXAMPLE 1-2

These Examples demonstrate the preparation of chewable spheroidal coated microcapsules according to the process of the present invention.

The spray congealed spheroidal microcapsule core was prepared having the composition set out in Table 1.

## TABLE 1

### SPRAY CONGEALED MICROCAPSULE CORE COMPOSITION

| INGREDIENT | AMOUNT | PERCENTAGE BY WEIGHT OF MICROCAPSULE CORE |
|---|---|---|
| Pseudoephedrine sulfate | 300g | 10.31 |
| Glyceryl Tristearate | 1185g | 40.72 |
| Carnauba Wax | 1185g | 40.72 |
| Acetylated monoglycerides | 150g | 5.16 |
| Fatty acid monoglycerides | 60g | 2.06 |
| Aluminum stearate | 30g | 1.03 |

The microcapsule core composition was prepared by mixing at low shear the glyceryl tristearate and carnauba wax in a steam jacketed tank maintained at a temperature of about 100°C. until a homogeneous mixture was obtained. A premix of medicament and emulsifying agent and the remaining ingredients was dispersed into the melted wax mixture and mixed to uniformly disperse the material in the mixture. The dispersion was screened through a U.S. standard mesh No. 80 screen and then fed into a heat controlled spray nozzle and spray congealed. The nozzle pressure was regulated to control the particle droplet size.

A quantity of 2910g of spray congealed microcapsules containing pseudoephedrine sulfate was obtained which was passed through a <20 mesh screen (greater than 99% of the microcapsules passed through the screen). The spray congealed microcapsules contained 10.31% pseudoephedrine sulfate, by weight.

The spray congealed microcapsule core was then spray coated with a first wax spray coating layer according to the method of the present invention. A quantity of 390g of pseudoephedrine sulfate microcapsule core material (<80 mesh) from Table 1 was coated with the first wax spray coating layer material having the composition set out in Table 2.

## TABLE 2

### FIRST WAX SPRAY COATING LAYER COMPOSITION

| INGREDIENT | AMOUNT | PERCENTAGE BY WEIGHT WAX COATING LAYER |
|---|---|---|
| Carnauba wax | 205g | 50 |
| Glyceryl Tristearate | 164g | 40 |
| Paraffin 150 | 20.5g | 5 |
| Fatty acid monoglycerides | 20.5g | 5 |

The wax coating layer material was loaded into a steam heated Hobart mixer at a temperature of about 92°C. and mixed until homogeneous. The pseudoephedrine sulfate microcapsule core material prepared above was then fluidized in a Versa-Glatt model GPCG 1 fluidized bed apparatus. The fluidized bed apparatus was then heated to the spraying-processing temperature. The pseudoephedrine sulfate microcapsules were then spray coated by heated air atomization with the first coating layer mixture until an approximate 100% weight gain was obtained. The first wax coated microcapsules were then removed from the apparatus.

A quantity of 800g of coated microcapsules having a first wax spray coating layer containing 5.02% pseudoephedrine sulfate was obtained.

The first wax spray coated microcapsules were then coated with a second oil coating layer of vegetable oil according to the process of the present invention. A quantity of 1.8103g of pseudoephedrine sulfate first wax spray coated microcapsules (<80 mesh) was coated with the second oil coating layer material having the composition set out in Table 3.

## TABLE 3

### SECOND OIL COATING LAYER COMPOSITION

| INGREDIENT | AMOUNT | PERCENTAGE BY WEIGHT SECOND COATING LAYER |
|---|---|---|
| Paramount fat C | 1.2g | 66.7% |
| Hard Palm Oil | 0.6g | 33.3% |

The Paramount fat C and hard palm oil were heated until melted. The pseudoephedrine sulfate microcapsule first wax spray coated material prepared above was then admixed into the melted second oil coating layer mixture to form a homogeneous mixture. The mixture was then incorporated into a nougat base (Example 1). A portion of the pseudoephedrine sulfate microcapsule first wax spray coated material prepared above, without a coating of the second oil coating layer mixture, was incorporated into a nougat base as a control sample (Example 2).

The nougat compositions were then compressed into cakes of approximately 8g each. These cakes were further divided into small samples to study the sustained release properties of the composition.

The sustained release properties (dissolution) of the coated microcapsules of Example 1 and the control microcapsules of Example 2 in nougat composition were measured by the paddle method (The United States Pharmacopeia XXI) at 100 rpm at 37°C. Each nougat was cut into 32 pieces and then introduced into the dissolution apparatus with 1000ml of distilled water. The dissolution of the medicament from the microcapsule samples was measured by High Pressure Liquid Chromatography using an ultraviolet detector. The results of the dissolution study were pooled and averaged and are set out in Table 4.

## TABLE 4

### DISSOLUTION STUDY

| TIME (hours) | EXAMPLE (% Dissolution by Weight) | |
|---|---|---|
| | 1 (invention) | 2 (control) |
| 1 | 29.70 | 37.07 |
| 2 | 41.93 | 68.68 |
| 4 | 57.86 | 77.85 |
| 6 | 69.58 | 81.53 |
| 8 | 73.70 | 82.0 |
| 12 | 78.71 | 84.38 |

Table 4 shows that the chewable spheroidal coated microcapsules having a second oil coating layer of a vegetable oil have improved delayed sustained release properties compared to spray coated microcapsules without a second oil coating layer.

The invention being thus described, it will be obvious that the same may be varied in many ways. Such variations are not to be regarded as a departure from the spirit and scope of the invention and all such modifications are intended to be included within the scope of the following claims.

The present invention is summarised by the following clauses, numbered 1 to 29, and is defined by the claims appended thereafter.

1. A chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, wherein the coated microcapsule comprises:
   (A) a microcapsule core comprising, in percentages by weight of the microcapsule core:
       (a) a medicament present in an amount from about 1% to about 40%;
       (b) an edible material having a melting point from about 25°C to about 100°C selected from the group consisting of (i) fatty acids having an iodine value from about 1 to about 10, (ii) natural waxes, (iii) synthetic waxes, and (iv) mixtures thereof, present in an amount from about 9.5% to about 98.5%;
       (c) an emulsifying agent present in an amount from about 0.5% to about 20%;
   (B) a first wax spray coating layer over the microcapsule core comprising, in percentages by weight of the first coating layer:

(a) an edible material having a melting point from about 25°C to about 100°C selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(C) a second oil coating layer over the first coated microcapsule core comprising an edible vegetable oil having a melting point in the range from about 25°C to about 90°C.

2. The coated microcapsule according to 1, wherein the medicament in the microcapsule core is present in an amount from about 1% to about 70%, by weight of the microcapsule core.

3. The coated microcapsule according to 1, wherein the edible material in the microcapsule core is present in an amount from about 29.5% to about 98.5%, by weight of the microcapsule core.

4. The coated microcapsule according to 1, wherein the emulsifying agent in the microcapsule core is present in an amount from about 5% to about 15%, by weight of the microcapsule core.

5. The coated microcapsule according to 1, wherein the edible material in the first wax spray coating layer is present in an amount from about 85% to about 99.5%, by weight of the first coating layer.

6. The coated microcapsule according to 1, wherein the emulsifying agent in the first wax spray coating layer is present in an amount from about 0.5% to about 15%, by weight of the first coating layer.

7. The coated microcapsule according to 1, wherein the medicament in the microcapsule core is a water-soluble medicament selected from the group consisting of dextromethorphan, dextromethorphan hydrobromide, pseudoephedrine, pseudoephedrine sulfate, pseudoephedrine hydrochloride, pseudoephedrine hydrobromide, chlorpheniramine maleate, guaifenesin, diphenhydramine hydrochloride, and mixtures thereof.

8. The coated microcapsule according to 1, wherein the edible material in the microcapsule core and in the first coating layer is selected from the group consisting of hydrogenated palm oil, hydrogenated castor oil, hydrogenated cottonseed oil, stearic acid, palmitic acid, and mixtures thereof.

9. The coated microcapsule according to 1, wherein the emulsifying agent in the microcapsule core and in the first coating layer is selected from the group consisting of glyceryl monostearate, acetylated monoglycerides, stearic acid, and mixtures thereof.

10. The coated microcapsule according to 1, wherein the edible vegetable oil in the second oil coating layer is selected from the group consisting of hard palm oil, partially hydrogenated castor oil, partially hydrogenated cotton seed oil, partially hydrogenated soybean oil, and a blend of partially hydrogenated palm kern oil, partially hydrogenated cotton seed oil, and partially hydrogenated soybean seed oil, and mixtures thereof.

11. The coated microcapsule according to 10, wherein the edible vegetable oil in the second oil coating layer is a mixture of hard palm oil, and a blend of partially hydrogenated palm kern oil, partially hydrogenated cotton seed oil and partially hydrogenated soybean seed oil, in a ratio of 2:1, respectively.

12. The coated microcapsule according to 1, wherein the weight ratio of microcapsule core to first wax spray coating layer to second oil coating layer is from about 1:1:0.5 to about 1:3:3, respectively.

13. The coated microcapsule according to 1, wherein

(a) a microcapsule core comprising the following ingredients:

| INGREDIENT | PERCENTAGE RANGE BY WEIGHT OF MICROCAPSULE CORE |
|---|---|
| Pseudoephedrine sulfate | 1-40 |
| Glyceryl Tristearate | 1-95.9 |
| Carnauba Wax | 1-95.9 |
| Acetylated monoglycerides | 1-10 |
| Fatty acid monoglycerides | 1-10 |
| Aluminum stearate | 0.1-5 |

(b) a first wax spray coating layer comprising the following ingredients:

| INGREDIENT | PERCENTAGE RANGE BY WEIGHT OF FIRST COATING LAYER |
|---|---|
| Carnauba wax | 1-97 |
| Glyceryl Tristearate | 1-97 |
| Paraffin 150 | 1-10 |
| Fatty acid monoglycerides | 1-10 |

(C) a second oil coating layer comprising the following ingredients:

| INGREDIENT | PERCENTAGE RANGE BY WEIGHT OF SECOND COATING LAYER |
|---|---|
| Blend of | 10-90 |
| Partially hydrogenated cotton seed oil | |
| Partially hydrogenated soybean seed oil | |
| Partially hydrogenated palm kern oil | |
| Palm Oil | 10-90 |

14. A method for preparing a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, which comprises the steps of:

(A) providing the following ingredients of the microcapsule core, in percentages by weight of the microcapsule core:

(a) a medicament present in an amount from about 1% to about 40%;

(b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%; and

(c) an emulsifying agent present in an amount from about 0.5% to about 20%; and

(B) mixing and heating the microcapsule core ingredients from step (A) to prepare a homogeneous mixture;

(C) feeding the mixture from step (B) into a heat controlled pressure spray nozzle and atomizing the mixture under controlled conditions such that spheroidal spray congealed core microcapsules under about 200 microns in diameter are formed;

(D) providing the following ingredients of the first wax spray coating layer, in percentages by weight of the first coating layer:

(a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(E) preparing a homogeneous mixture of the first coating layer ingredients from step (D);

(F) coating the spray congealed core microcapsules from step (C) with the first coating layer mixture from step (E) in a fluid bed dryer by suspending the microcapsule cores in a stream of air passing through a zone of the first coating layer mixture under controlled conditions such that spheroidal first coated microcapsules under about 600 microns in diameter are formed;

(G) providing a second oil coating layer comprising an edible vegetable oil having a melting point in the range from about 25°C. to about 90°C.;

(H) mixing and melting the second oil coating layer ingredients from step (G) to form a homogeneous mixture; and

(I) admixing the first coated microcapsules from step (F) with the melted second layer mixture from step (H) to prepare a homogeneous mixture of chewable spheroidal coated microcapsules under about 850 microns in diameter.

15. The method according to 14, wherein the medicament in the microcapsule core is present in an amount from about 1% to about 70%, by weight of the microcapsule core.

16. The method according to 14, wherein the edible material in the microcapsule core is present in an amount from about 29.5% to about 98.5%, by weight of the microcapsule core.

17. The method according to 14, wherein the emulsifying agent in the microcapsule core is present in an amount from about 5% to about 15%, by weight of the microcapsule core.

18. The method according to 14, wherein the edible material in the first wax spray coating layer is present in an amount from about 85% to about 99.5%, by weight of the first coating layer.

19. The method according to 14, wherein the emulsifying agent in the first wax spray coating layer is present in an amount from about 0.5% to about 15%, by weight of the first coating layer.

20. The method according to 14, wherein the medicament in the microcapsule core is a water-soluble medicament selected from the group consisting of dextromethorphan, dextromethorphan hydrobromide, pseudoephedrine, pseudoephedrine sulfate, pseudoephedrine hydrochloride, pseudoephedrine hydrobromide, chlorpheniramine maleate, guaifenesin, diphenhydramine hydrochloride, and mixtures thereof.

21. The method according to 14, wherein the edible material in the microcapsule core and in the first coating layer is selected from the group consisting of hydrogenated palm oil, hydrogenated castor oil, hydrogenated cottonseed oil, stearic acid, palmitic acid, and mixtures thereof.

22. The method according to 14, wherein the emulsifying agent in the microcapsule core and in the first coating layer is selected from the group consisting of glyceryl monostearate, acetylated monoglycerides, stearic acid, and mixtures thereof.

23. The method according to 14, wherein the edible vegetable oil in the second oil coating layer is selected from the group consisting of hard palm oil, partially hydrogenated castor oil, partially hydrogenated cotton seed oil, partially hydrogenated soybean oil, and a blend of partially hydrogenated palm kern oil, partially hydrogenated cotton seed oil, and partially hydrogenated soybean seed oil, and mixtures thereof.

24. The method according to 14, wherein the weight ratio of microcapsule core to first wax spray coating layer to second oil coating layer is from about 1:1:0.5 to about 1:3:3, respectively.

25. A chewable sustained release medicated composition which comprises a pharmaceutically acceptable carrier and a therapeutically effective amount of a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, wherein the coated microcapsule comprises:

(A) a microcapsule core comprising, in percentages by weight of the microcapsule core:

(a) a medicament present in an amount from about 1% to about 40%;

(b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%;

(c) an emulsifying agent present in an amount from about 0.5% to about 20%;

(B) a first wax spray coating layer over the microcapsule core comprising, in percentages by weight of the first coating layer:

(a) an edible material having a melting point from about 25°C to abut 100°C selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(C) a second oil coating layer over the first coated microcapsule core comprising an edible vegetable oil having a melting point in the range from about 25°C to about 90°C.

26. The sustained release medicated composition according to 25, wherein the coated microcapsules are present in the medicated composition in an amount from about 5% to about 50%, by weight of the medicated composition.

27. The sustained release medicated composition according to 25, wherein the pharmaceutically acceptable carrier is nougat.

28. The sustained release medicated composition according to 25, wherein the coated microcapsule comprises:

(a) a microcapsule core comprising the following ingredients:

| INGREDIENT | PERCENTAGE RANGE BY WEIGHT OF MICROCAPSULE CORE |
|---|---|
| Pseudoephedrine sulfate | 1-40 |
| Glyceryl Tristearate | 1-95.9 |
| Carnauba Wax | 1-95.9 |
| Acetylated monoglycerides | 1-10 |
| Fatty acid monoglycerides | 1-10 |
| Aluminum stearate | 0.1-5 |

(b) a first wax spray coating layer comprising the following ingredients:

| INGREDIENT | PERCENTAGE RANGE BY WEIGHT OF FIRST COATING LAYER |
|---|---|
| Carnauba wax | 1-97 |
| Glyceryl Tristearate | 1-97 |
| Paraffin 150 | 1-10 |
| Fatty acid monoglycerides | 1-10 |

(C) a second oil coating layer comprising the following ingredients:

| INGREDIENT | PERCENTAGE RANGE BY WEIGHT OF SECOND COATING LAYER |
|---|---|
| Blend of | 10-90 |
| Partially hydrogenated cotton seed oil | |
| Partially hydrogenated soybean seed oil | |
| Partially hydrogenated palm kern oil | |
| Palm Oil | 10-90 |

29. A method for preparing a chewable sustained release medicated composition which comprises a pharmaceutically acceptable carrier and a therapeutically effective amount of a chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, which comprises the steps of:

(A) providing the following ingredients of the microcapsule core, in percentages by weight of the microcapsule core:

   (a) a medicament present in an amount from about 1% to about 40%;

   (b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%; and

   (c) an emulsifying agent present in an amount from about 0.5% to about 20%; and

(B) mixing and heating the microcapsule core ingredients from step (A) to prepare a homogeneous mixture;

(C) feeding the mixture from step (B) into a heat controlled pressure spray nozzle and atomizing the mixture under controlled conditions such that spheroidal spray congealed core microcapsules under about 200 microns in diameter are formed;

(D) providing the following ingredients of the first wax spray coating layer, in percentages by weight of the first coating layer:

   (a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(E) preparing a homogeneous mixture of the first coating layer ingredients from step (D);

(F) coating the spray congealed core microcapsules from step (C) with the first coating layer mixture from step (E) in a fluid bed dryer by suspending the microcapsule cores in a stream of air passing through a zone of the first coating layer mixture under controlled conditions such that spheroidal first coated microcapsules under about 600 microns in diameter are formed;

(G) providing a second oil coating layer comprising an edible vegetable oil having a melting point in the range from about 25°C. to about 90°C.;

(H) mixing and melting the second oil coating layer ingredients from step (G) to form a homogeneous mixture;

(I) admixing the first coated microcapsules from step (F) with the melted second layer mixture from step (H) to prepare a homogeneous mixture of chewable spheroidal coated microcapsules under about 850 microns in diameter; and

(J) admixing the microcapsules from step (I) having a first coating layer and a second coating layer with the pharmaceutical carrier to form a chewable sustained release medicated composition.

## Claims

1. A chewable spheroidal coated microcapsule under about 850 microns in diameter which comprises at least one spray congealed spheroidal microcapsule core under about 200 microns in diameter, a first wax spray coating layer over the core, and a second oil coating layer over the first coated core, wherein the coated microcapsule comprises:

   (A) a microcapsule core comprising, in percentages by weight of the microcapsule core:

   (a) a medicament present in an amount from about 1% to about 40%;

   (b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%;

   (c) an emulsifying agent present in an amount from about 0.5% to about 20%;

   (B) a first wax spray coating layer over the microcapsule core comprising, in percentages by weight of the first coating layer:

   (a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%, and preferably present in an amount from about 80% to about 99.5%;

   (b) an emulsifying agent present in an amount from about 0.5% to about 20%; and

   (C) a second oil coating layer over the first coated microcapsule core comprising an edible vegetable oil having a melting point in the range from about 25o C. to about 90o C.

2. The coated microcapsule according to claim 1, wherein the medicament in the microcapsule core is present in an amount from about 1% to about 70%, by weight of the microcapsule core.

3. The coated microcapsule according to either of the preceding claims, wherein the edible material in the microcapsule core is present in an amount from about 29.5% to about 98.5%, by weight of the microcapsule core, and is selected from the group consisting of hydrogenated palm oil, hydrogenated castor oil, hydrogenated cottonseed oil, stearic acid, palmitic acid, and mixtures thereof.

4. The coated microcapsule according to any preceding claim, wherein the emulsifying agent in the microcapsule core is present in an amount from about 5% to about 15%, by weight of the microcapsule core, and is selected from the group consisting of glyceryl monostearate, acetylated monoglycerides, stearic acid, and mixtures thereof.

5. The coated microcapsule according to any preceding claim, wherein the edible material in the first wax spray coating layer is present in an amount from about 85% to about 99.5%, by weight of the first coating layer, and is selected from the group consisting of hydrogenated palm oil, hydrogenated castor oil, hydrogenated cottonseed oil, stearic acid, palmitic acid, and mixtures thereof.

6. The coated microcapsule according to any preceding claim, wherein the emulsifying agent in the first wax spray coating layer is present in an amount from about 0.5% to about 15%, by weight of the first coating layer, and is selected from the group consisting of glyceryl monostearate, acetylated monoglycerides, stearic acid, and mixtures thereof.

7. The coated microcapsule according to any preceding claim, wherein the medicament in the microcapsule core is a water-soluble medicament selected from the group consisting of dextromethorphan, dextromethorphan hydrobromide, pseudoephedrine, pseudoephedrine sulfate, pseudoephedrine hydrochloride, pseudoephedrine hydrobromide, chlorpheniramine maleate, guaifenesin, diphenhydramine hydrochloride, and mixtures thereof.

8. The coated microcapsule according to any preceding claim, wherein the edible vegetable oil in the second oil coating layer is selected from the group consisting of hard palm oil, partially hydrogenated castor oil, partially hydrogenated cotton seed oil, partially hydrogenated soybean oil, and a blend of partially hydrogenated palm kern oil, partially hydrogenated cotton seed oil, and partially hydrogenated soybean seed oil, and preferably is a mixture of hard palm oil, and a blend of partially hydrogenated palm kern oil, partially hydrogenated cotton seed oil and partially hydrogenated soybean seed oil, in a ratio of 2:1, respectively.

9. The coated microcapsule according to any preceding claim, wherein the weight ratio of microcapsule core to first wax spray coating layer to second oil coating layer is from about. 1:1:0.5 to about 1:3:3, respectively.

10. The coated microcapsule according to claim 1, wherein
    (a) a microcapsule core comprising the following ingredients:

| INGREDIENT | PERCENTAGE RANGE BY WEIGHT OF MICROCAPSULE CORE |
|---|---|
| Pseudoephedrine sulfate | 1-40 |
| Glyceryl Tristearate | 1-95.9 |
| Carnauba Wax | 1-95.9 |
| Acetylated monoglycerides | 1-10 |
| Fatty acid monoglycerides | 1-10 |
| Aluminum stearate | 0.1-5 |

    (b) a first wax spray coating layer comprising the following ingredients:

| INGREDIENT | PERCENTAGE RANGE BY WEIGHT OF FIRST COATING LAYER |
|---|---|
| Carnauba wax | 1-97 |
| Glyceryl Tristearate | 1-97 |
| Paraffin 150 | 1-10 |
| Fatty acid monoglycerides | 1-10 |

    (C) a second oil coating layer comprising the following ingredients:

| INGREDIENT | PERCENTAGE RANGE BY WEIGHT OF SECOND COATING LAYER |
|---|---|
| Blend of | 10-90 |
| Partially hydrogenated cotton seed oil | |
| Partially hydrogenated soybean seed oil | |
| Partially hydrogenated palm kern oil | |
| Palm Oil | 10-90 |

11. A method for preparing a chewable spheroidal coated microcapsule of any preceding claim, which method comprises the steps of:

(A) providing the following ingredients of the microcapsule core, in percentages by weight of the microcapsule core:

(a) a medicament present in an amount from about 1% to about 40%;

(b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%; and

(c) an emulsifying agent present in an amount from about 0.5% to about 20%; and

(B) mixing and heating the microcapsule core ingredients from step (A) to prepare a homogeneous mixture;

(C) feeding the mixture from step (B) into a heat controlled pressure spray nozzle and atomizing the mixture under controlled conditions such that spheroidal spray congealed core microcapsules under about 200 microns in diameter are formed;

(D) providing the following ingredients of the first wax spray coating layer, in percentages by weight of the first coating layer:

(a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(E) preparing a homogeneous mixture of the first coating layer ingredients from step (D);

(F) coating the spray congealed core microcapsules from step (C) with the first coating layer mixture from step (E) in a fluid bed dryer by suspending the microcapsule cores in a stream of air passing through a zone of the first coating layer mixture under controlled conditions such that spheroidal first coated microcapsules under about 600 microns in diameter are formed;

(G) providing a second oil coating layer comprising an edible vegetable oil having a melting point in the range from about 25°C. to about 90°C.;

(H) mixing and melting the second oil coating layer ingredients from step (G) to form a homogeneous mixture; and

(I) admixing the first coated microcapsules from step (F) with the melted second layer mixture from step (H) to prepare a homogeneous mixture of chewable spheroidal coated microcapsules under about 850 microns in diameter.

12. A chewable sustained release medicated composition which comprises a pharmaceutically acceptable carrier and the chewable spheroidal coated microcapsule of any one of claims 1 to 10, present in an amount of from about 5% to about 50%, by weight of the medicated composition.

13. The sustained release medicated composition according to claim 12, wherein the pharmaceutically acceptable carrier is nougat.

14. A method for preparing a chewable sustained release medicated composition of either of claims 12 or 13, which method comprises the steps of:

(A) providing the following ingredients of the microcapsule core, in percentages by weight of the microcapsule core:

(a) a medicament present in an amount from about 1% to about 40%;

(b) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 9.5% to about 98.5%; and

(c) an emulsifying agent present in an amount from about 0.5% to about 20%; and

(B) mixing and heating the microcapsule core ingredients from step (A) to prepare a homogeneous mixture;

(C) feeding the mixture from step (B) into a heat controlled pressure spray nozzle and atomizing the mixture under controlled conditions such that spheroidal spray congealed core microcapsules under about 200 microns in diameter are formed;

(D) providing the following ingredients of the first wax spray coating layer, in percentages by weight of the first coating layer:

(a) an edible material having a melting point from about 25°C. to about 100°C. selected from the group consisting of (i) fatty acids having an iodine value from about 1 to about 10, (ii) natural waxes, (iii) synthetic waxes, and (iv) mixtures thereof, present in an amount from about 60% to about 99.5%;

(b) an emulsifying agent present in an amount from about 0.5% to about 20%; and

(E) preparing a homogeneous mixture of the first coating layer ingredients from step (D);

(F) coating the spray congealed core microcapsules from step (C) with the first coating layer mixture from step (E) in a fluid bed dryer by suspending the microcapsule cores in a stream of air passing through a zone of the first coating layer mixture under controlled conditions such that spheroidal first coated microcapsules under about 600 microns in diameter are formed;

(G) providing a second oil coating layer comprising an edible vegetable oil having a melting point in the range from about 25°C. to about 90°C.;

(H) mixing and melting the second oil coating layer ingredients from step (G) to form a homogeneous mixture;

(I) admixing the first coated microcapsules from step (F) with the melted second layer mixture from step (H) to prepare a homogeneous mixture of chewable spheroidal coated microcapsules under about 850 microns in diameter; and

(J) admixing the microcapsules from step (I) having a first coating layer and a second coating layer with the pharmaceutical carrier to form a chewable sustained release medicated composition.

European Patent
Office

**EUROPEAN SEARCH REPORT**

Application Number

| DOCUMENTS CONSIDERED TO BE RELEVANT | | | EP 91810677.4 |
|---|---|---|---|
| Category | Citation of document with indication, where appropriate, of relevant passages | Relevant to claim | CLASSIFICATION OF THE APPLICATION (Int. Cl. 5) |
| P,X | EP - A - 0 421 582 (WARNER-LAMBERT COMPANY) * Abstract; claims 1,5,17, 27,35-40 * -- | 1,7, 11-14 | A 61 K 9/50 A 61 K 9/52 A 61 K 9/68 |
| P,X | EP - A - 0 421 581 (WARNER-LAMBERT COMPANY) * Abstract; claims 1,3,11, 17,22-25,34 * -- | 1,7, 11,14 | |
| P,A | EP - A - 0 413 533 (EURAND AMERICA INCORPORATED) * Abstract; claims 1,6 * -- | 1,11, 14 | |
| A | WO - A - 88/03 795 (MEHTA) * Abstract; claims 1,10,11, 15 * ---- | 1,7, 11,14 | |
| | | | TECHNICAL FIELDS SEARCHED (Int. Cl.5) A 61 K |

The present search report has been drawn up for all claims

| Place of search | Date of completion of the search | Examiner |
|---|---|---|
| VIENNA | 19-12-1991 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)